## Europäisches Patentamt

## European Patent Office

## Office européen des brevets

(19)

(11) Veröffentlichungsnummer : **0 098 590**
**B1**

(12) **EUROPÄISCHE PATENTSCHRIFT**

(45) Veröffentlichungstag der Patentschrift :
01.02.89

(21) Anmeldenummer : 83106566.9

(22) Anmeldetag : 05.07.83

(51) Int. Cl.⁴ : **G 01 N 33/543**, G 01 N 33/541

(54) **Immunchemisches Messverfahren.**

(30) Priorität : 05.07.82 DE 3225027

(43) Veröffentlichungstag der Anmeldung :
18.01.84 Patentblatt 84/03

(45) Bekanntmachung des Hinweises auf die Patenterteilung : 01.02.89 Patentblatt 89/05

(84) Benannte Vertragsstaaten :
AT BE CH DE FR GB IT LI LU NL SE

(56) Entgegenhaltungen :
EP--A-- 0 008 473
EP--A-- 0 064 318
CA--A-- 1 123 336
GB--A-- 2 001 171
GB--A-- 2 074 727
GB--A-- 2 084 317

(73) Patentinhaber : **BOEHRINGER MANNHEIM GMBH**
**Patentabteilung, Abt. E Sandhofer Strasse 112-132**
**Postfach 31 01 20**
**D-6800 Mannheim 31 Waldhof (DE)**

(72) Erfinder : **Tanswell, Paul, Dr. phil.**
**Eichendorffweg 14**
**D-7958 Laupheim 1 Obersulmetingen (DE)**
Erfinder : **Baier, Manfred, Dr. rer. nat.**
**Tiefentalweg 10**
**D-8124 Seeshaupt (DE)**
Erfinder : **Lenz, Helmut, Dr. rer. nat.**
**Waldschmidtstrasse 7**
**D-8132 Tutzing (DE)**

(74) Vertreter : **Weickmann, Heinrich, Dipl.-Ing. et al**
**Patentanwälte Dipl.-Ing. H.Weickmann Dipl.-Phys.Dr.**
**K.Fincke Dipl.-Ing. F.A.Weickmann Dipl.-Chem. B.**
**Huber Dr.-Ing. H. Liska Dipl.-Phys.Dr. J. Prechtel**
**Postfach 860820**
**D-8000 München 86 (DE)**

## Beschreibung

Die Erfindung betrifft ein immunchemisches Verfahren zur Bestimmung eines mindestens bifunktionellen Liganden (Antigen) in einer flüssigen Probe.

Die empfindliche Bestimmung von polyvalenten Antigenen (Peptide, Proteine) unter Verwendung von zwei Antikörpern, die gegen unterschiedliche Antigendeterminanten gerichtet sind, ist bekannt als 2-Site immunoradiometischer bzw. immunoenzymomotrischer Assay z. B. aus J. Clin. Chem. Clin. Biochem. 18 (1980) 197-208. Am gebräuchlichsten ist die Durchführung dieses bekannten Bestimmungsverfahrens, indem zuerst das zu bestimmende Antigen mit einem ersten Antikörper, der in fester Phase vorliegt durch Bindung an ein geeignetes Trägermaterial wie Sepharose, Agarose, Kunststoffröhrchen oder dergleichen, inkubiert wird. Während dieser ersten Inkubation bindet der erste Antikörper, der in der Regel in großem Überschuß vorhanden sein muß, mit einer Antigendeterminante des zu bestimmenden Antigens. Dann wird üblicherweise die Probeflüssigkeit von der festen Phase getrennt, um Störungen durch nicht spezifische Substanzen wie Humanserumprotein bzw. durch kreuzreagierende Antigene in der anschließenden zweiten Inkubation auszuschalten. Dann wird eine bestimmte Menge eines zweiten markierten Antikörpers in flüssiger Phase mit der festen Phase inkubiert. Dabei wird die Spezifität des zweiten Antikörpers bevorzugt so gewählt, daß sie gegen eine andere Determinante des zu bestimmenden Antigens gerichtet ist als der erste Antikörper, um eine Konkurrenz zwischen den beiden Antikörpern um die selben Bindungsstellen an dem zu bestimmenden Antigen auszuschließen, da hierdurch die Testempfindlichkeit beeinträchtigt würde.

Während dieser zweiten Inkubation reagiert der markierte zweite Antikörper, der ebenfalls regelmäßig im Überschuß vorliegt, mit sämtlichen Bindungsstellen an Molekülen des zu bestimmenden Antigens. Nach der zweiten Inkubation kann die Aktivität der Markierungssubstanz entweder an der festen Phase oder im Überstand gemessen werden. Üblicherweise erfolgt die Messung dabei an der festen Phase nach Auswaschung der flüssigen Phase. Im gunstigsten Fall verhält sich hierbei die bestimmte Aktivität fast proportional zur Menge des zu bestimmenden Antigens.

Dieses 2-Schritt-Sandwich-Verfahren hat den Vorteil, daß eventuell kreuzreagierende Antigene bereits bei der ersten Phasentrennung entfernt werden. Dies ist von besonderer Bedeutung bei Testsystemen, bei denen die beiden Antikörper wegen der Empfindlichkeitsanforderungen wirklich unterschiedliche Spezifität besitzen müssen. In diesem Fall besteht die hohe Anforderung an die Spezifität nur für einen der beiden Antikörper, der andere muß dann nicht absolut spezifisch gegenüber kreuzreagierenden Antigenen sein.

Dieses Verfahren hat jedoch drei wesentliche Nachteile.

1. Im ersten Inkubationsschritt liegt ein Reaktant in fester Phase vor, der andere in Lösung. Die Geschwindigkeitskonstante der Reaktion ist damit geringer als wenn sich beide Reaktionsteilnehmer (Antigen und erster Antikörper) in Lösung befänden. Es muß jedoch so lange inkubiert werden, bis alle Antigenmoleküle an die feste Phase gebunden vorliegen, da nicht gebundenes Antigen bei der Phasentrennung entfernt würde und damit eine Verfälschung des Resultats zur Folge hätte.

2. Die Bindung eines für das Antigen spezifisch bindefähigen Antikörpers an eine Festphase erfordert wegen der geringen Bindungsausbeuten relativ große Mengen an erstem Antikörper, der meist eine sehr kostbare Substanz ist, insbesondere wenn das Antiserum aus Spezifitätsgründen in Kleintieren, wie Kaninchen, Meerschweinchen und dergleichen, gewonnen werden muß. Außerdem können bei der Bindungsreaktion an die Festphase Affinitätsverschlechterungen am Antikörper entstehen, was sich nachteilig auf die Empfindlichkeit auswirkt. ·

3. Die Gewinnung von zwei Antikörpern unterschiedlicher Spezifität gegen das gleiche Antigen ist in der Regel aufwendig und vielfach nur begrenzt möglich, beispielsweise indem man zwei verschiedene Tierspezies immunisiert oder indem man die Antikörper-Populationen aus einem Tier durch Immunadsorption an dem zu bestimmenden Antigen in geeignete Fragmente aufzutrennen versucht.

Es sind bereits verschiedene Versuche bekannt, die obigen Mängel zu beheben. So wird gemäß US-PS 40 98 876 die erste Inkubation des Antigens mit Isotopen-markiertem gelöstem Antikörper durchgeführt. Erst danach wird der Festphasengebundene Antikörper hinzugefügt und die zweite Inkubation vorgenommen. Hier ist nur noch eine Phasentrennung erforderlich, was eine gewisse Erhöhung der Empfindlichkeit und Praktikabilität ergibt. Das Spezifitätsproblem bleibt jedoch ungelöst, da beide Antikörper hochspezifisch gegen das Antigen gerichtet sein müssen. Außerdem wird in Anwesenheit größerer Mengen kreuzreagierender Antigene die scheinbare Konzentration des zu bestimmenden Antigens bei einer etwaigen Unspezifität des zweiten Antikörpers erhöht, bei Unspezifität des ersten Antikörpers herabgesetzt.

In der DE-OS 29 25 565 wird ein Verfahren beschrieben, bei dem der in fester Phase vorliegende erste Antikörper und der markierte gelöste zweite Antikörper gleichzeitig mit dem Antigen inkubiert werden. Hier bestehen die gleichen Probleme wie bei dem zuvor genannten Verfahren. Außerdem bleiben auch die oben für das Basisverfahren beschriebenen Nachteile 2 und 3 ungelöst.

Aus der offengelegten japanischen Patentanmeldung 123 802 vom 06.10.1978 ist schließlich ein Verfahren bekannt, bei welchem ein Festphasen-Antikörper verwendet wird, der spezifisch gegen den zweiten Antikörper gerichtet ist, welcher mit dem zu messenden Antigen bindefähig ist. Hier wird die Bindung eines gegen das Antigen gerichteten Antikörpers an die feste Phase vermieden. Jedoch wird die Empfindlichkeit herabgesetzt, da nur ein einziger mit dem Antigen spezifisch bindefähiger Antikörper verwendet wird. Der Konzentrationsnachweis des zu bestimmenden Antigens erfolgt über die kompetetive Reaktion zwischen dem Antigen und einer bestimmten Menge an Isotopen-markiertem Antigen mit dem spezifischen Antikörper.

Schließlich ist aus Clin. Chem. 27/6, (1981) 823-827 eine radiometrische Bestimmungsmethode für Creatinkinase MB-Isoenzym bekannt, bei welcher in einem ersten Inkubationsschritt das Antigen gleichzeitig mit einem unmarkierten und mit einem markierten Antikörper unterschiedlicher Spezifität und aus unterschiedlichen Tierspezies umgesetzt wird. Im zweiten Schritt wird dann ein in fester Phase vorliegender Antikörper mit Spezifität gegen den unmarkierten Antikörper des ersten Inkubationsschrittes zugesetzt. Der Nachteil dieses Verfahrens besteht wiederum darin, daß zwei verschiedene für das Antigen spezifische Antikörper und drei verschiedene Tierspezies benötigt werden.

Der Erfindung liegt daher die Aufgabe zugrunde, einen oder mehrere der oben geschilderten Nachteile des Basisverfahrens besser zu vermeiden als die bisher bekannten Verfahren.

Diese Aufgabe wird erfindungsgemäß gelöst durch ein Verfahren zur Bestimmung eines polyvalenten Antigens durch Inkubation mit drei verschiedenen Rezeptoren, von denen der erste und der dritte in flüssiger Phase vorliegen und mit dem Antigen bindefähig sind, der zweite Rezeptor in fester Phase vorliegt und mit dem ersten Rezeptor bindefähig ist und der dritte Rezeptor eine Markierung trägt und mit dem ersten und zweiten Rezeptor nicht kreuzreagiert, Trennung der festen von der flüssigen Phase und Messung der Markierung in einer der Phasen, welches dadurch gekennzeichnet ist, daß man das Antigen entweder mit allen drei Rezeptoren oder mit dem ersten und zweiten Rezeptor gleichzeitig inkubiert, dann die Phase trennt, gegebenenfalls die feste Phase mit dem dritten Rezeptor inkubiert und erneut die Phasen trennt.

Als Rezeptoren werden im Rahmen der Erfindung entweder spezifisch bindefähige komplette Antikörper, Antikörperfragmente oder Konjugate von Antikörpern oder Antikörperfragmenten mit Haptenen verwendet. Vorzugsweise wird als erster Rezeptor ein kompletter Antikörper oder ein kovalent mit einem Hapten verbundener Antikörper verwendet.

Als zweiter Rezeptor wird vorzugsweise ein Antikörper verwendet, welcher nur mit einem Teil des ersten Rezeptors bindefähig ist, besonders bevorzugt ein Antikörper, der mit dem Fc-Teil des ersten Rezeptors bindefähig ist. Alternativ läßt sich als zweiter Rezeptor auch ein Antikörper verwenden, der mit dem Haptenteil des ersten Rezeptors oder dem Fab-Teil oder mit dem gesamten ersten Rezeptor spezifisch bindefähig ist.

Der dritte Rezeptor darf mit dem zweiten Rezeptor nicht kreuzreagieren.

Bevorzugt verwendet man einen dritten Rezeptor, welcher aus einem Teil, z. B. einem Fragment, des ersten Rezeptors besteht und daher aus der gleichen Tierspezies wie der erste Rezeptor gewonnen wird. Dies gilt auch dann, wenn der erste Rezeptor aus einem Antikörper besteht, der ein Hapten trägt und der zweite Rezeptor nur mit diesem Hapten bindefähig ist. Besonders bevorzugt ist der dritte Rezeptor ein Fab-Fragment des ersten Rezeptors. Dies hat den Vorteil, daß nur zwei Tierspezies für die drei Rezeptoren benötigt werden. Wenn der zweite Rezeptor nur mit dem Hapten bindefähig ist, wird sogar nur eine Tierspezies für alle drei Rezeptoren benötigt.

Der dritte Rezeptor, der in bekannter Menge eingesetzt wird, ist in der dem Fachmann bekannten Weise markiert. Bevorzugt erfolgt die Markierung durch Kupplung mit einem Enzym, einer fluoreszierenden, chemilumineszierenden oder radioaktiven Substanz. Verfahren zur Markierung von derartigen Rezeptoren sind dem Fachmann bekannt, z. B. aus Clin. Chim. Acta 81 (1977) 1-40, und bedürfen hier keiner weiteren Erläuterung.

Das Verfahren der Erfindung kann so durchgeführt werden, daß die das zu bestimmende, mindestens zweiwertige Antigen enthaltende Probe mit allen drei Rezeptoren gleichzeitig inkubiert wird. In diesem Fall trennt man nach der Inkubation die feste und die flüssige Phase und bestimmt die Menge an markiertem dritten Rezeptor entweder in der flüssigen oder vorzugsweise in der festen Phase.

Gemäß einer bevorzugten Ausführungsform der Erfindung inkubiert man jedoch die antigenhaltige Probe zuerst mit dem ersten und dem zweiten Rezeptor, trennt danach die Phasen und inkubiert nur die abgetrennte feste Phase mit dem dritten Rezeptor in einem zweiten Inkubationsschritt. Danach wird wieder die feste von der flüssigen Phase getrennt und wie oben beschrieben, in einer der Phasen die Menge an markiertem dritten Rezeptor gemessen. Bei dieser zweistufigen Inkubation genügt es, wenn nur der erste oder der dritte Rezeptor spezifisch bindefähig mit dem Antigen ist, der jeweils andere Rezeptor kann auch mit anderen Antigenen bindefähig, d. h. unspezifisch sein. Bei der einstufigen Inkubation, also mit allen drei Rezeptoren, müssen der erste und der dritte Rezeptor spezifisch bindefähig sein.

Die Bindung des in fester Phase vorliegenden zweiten Rezeptors an ein unlösliches Trägermaterial kann nach den üblichen, dem Fachmann bekannten Methoden zur Fixierung biologisch aktiver Proteine an feste Trägersubstanzen vorgenommen werden. Sowohl eine kovalente als auch eine adsorptive Bindung ist geeignet. Bevorzugt wird jedoch, wegen der hierbei erzielbaren höheren Ausbeute und der vereinfachten Arbeitsweise, eine lediglich adsorptive Bindung, beispielsweise an Kunststoff. Als besonders geeignet erwiesen sich dabei Reagenzgläschen aus Polystyrol und ähnlichen Kunststoffen, die adsorptiv an der Innenoberfläche mit dem zweiten Rezeptor beschichtet sind. Die erste und gegebenenfalls zweite Inkubation wird dann in diesem Röhrchen oder anders geformten Behälter durchgeführt und zur Phasentrennung genügt es, die flüssige Phase aus dem Röhrchen zu entfernen, beispielsweise durch Absaugen und dergleichen. Nach Waschen des Röhrchens kann dann unmittelbar auch die Messung der Markierung darin vorgenommen werden, insbesondere wenn die Markierung mit einem Enzym erfolgt ist. Es genügt dann, einfach die Aktivität des Markierungsenzyms, beispielsweise Peroxidase oder ß-Galactosidase, in der hierfür allgemein bekannten Weise zu messen. Die gemessene Enzymaktivität ist dann ein Maß für die Menge an zu bestimmenden polyfunktionellen Antigen. Als Festphasenträger für den zweiten Rezeptor kommen aber auch teilchenförmige Substanzen, z. B. Ionenaustauscher, Molekularsiebmaterialien, Glaskörperchen, Kunststoffschläuche und dergleichen in Betracht.

Falls die Markierung nicht mit einem Enzym sondern mit einer radioaktiven Substanz, einem Isotop, einer fluoreszierenden oder chemilumineszierenden Substanz erfolgt, so wird auch hier die Bestimmung nach einer der dem Fachmann wohl bekannten Methoden vorgenommen. Eine Beschreibung dieser Meßmethoden erübrigt sich daher hier.

Überraschenderweise wurde gefunden, daß erfindungsgemäß eine deutlich höhere Empfindlichkeit als bei der üblichen 2-Schritt-Sandwich-Methode erreicht wird. Außerdem kann der erste Rezeptor in wesentlich geringerer Konzentration eingesetzt werden als dies möglich wäre, wenn der erste Rezeptor in fester Phase vorliegen würde.

Wird dabei gemäß der bevorzugten Ausführungsform der Erfindung ein gegen den Fc-Teil des ersten Rezeptors oder gegen das Hapten, welches mit dem ersten Rezeptor gekuppelt ist, gerichteter zweiter Rezeptor verwendet, so läßt sich hierbei eine weitere Steigerung der Empfindlichkeit erzielen, da eine eventuelle sterische Behinderung der Bindereaktion des Antigens mit der Bindungsstelle des ersten Rezeptors vermieden wird. Dies ist ein besonderer Vorteil, wenn Antigene mit besonders geringer Konzentration bestimmt werden müssen, wie dies z. B. bei der Bestimmung von Thyreotropin im Serum der Fall ist.

Wird erfindungsgemäß als dritter Rezeptor ein Fab-Fragment des ersten Rezeptors verwendet, so stammen, wie ohne weiteres ersichtlich, Rezeptor 1 und Rezeptor 3 vorzugsweise von der gleichen Tierspezies, da Rezeptor 2 ja nur Determinanten gegen den Fc-Teil des Antikörpers oder gegen das mit diesem gekuppelte Hapten aufweist.

Ein weiterer Gegenstand der Erfindung ist ein Trockenreagenz zur Bestimmung eines polyvalenten Antigens, welches dadurch gekennzeichnet ist, daß es zwei verschiedene lösliche, mit dem Antigen bindefähige Rezeptoren, von denen einer eine Markierung trägt, und einen nur mit dem nichtmarkierten löslichen Rezeptor bindefähigen unlöslichen Rezeptor enthält und der markierte Rezeptor einen Teil des nichtmarkierten löslichen Rezeptors enthält, wobei der unlösliche Rezeptor und der unmarkierte lösliche Rezeptor physikalisch getrennt vorliegen.

Die physikalische Trennung kann z. B. erfolgen, indem der unlösliche Rezeptor und der unmarkierte lösliche Rezeptor stufenweise nacheinander hydrophilisiert werden unter Aufbringung einer inerten Zwischenschicht, die beispielsweise aus Zucker bestehen kann, und auf die Schicht des zuerst lyophilisierten Akzeptors aufgebracht wird. So ist es beispielsweise möglich, den zweiten Rezeptor zuerst an der Innenwand eines Kunststoffröhrchens durch Lyophilisation unlöslich zu machen, darüber eine inerte Zwischenschicht, beispielsweise aus Rohrzucker, aufzulyophilisieren und dann den ebenfalls lyophilisierten unmarkierten löslichen Rezeptor, den ersten Rezeptor, zuzugeben. Der dritte, markierte lösliche Rezeptor kann dabei in Mischung mit dem ersten Rezeptor oder getrennt davon zugesetzt werden. Als inerte Zwischenschicht können außer Kohlehydraten auch leicht lösliche Polymere, beispielsweise in Form einer Folie aus einem wasserlöslichen Polymer, verwendet werden.

Die Erfindung eignet sich zur Bestimmung aller Antigene mit wenigstens zwei Antigendeterminanten. Beispiele hierfür sind Thyreotropin (TSH), 1-Fetoprotein (AFP), hCG, carcinoembryonales Antigen, luteinisierendes Hormon (LH), follikelstimulierendes Hormon (FSH), $\beta_2$-Microglobulin, saure Prostataphosphatase, Prolactin, Ferritin und Insulin.

Bei der Erfindung kann Rezeptor 1 homogen mit dem Antigen reagieren. Diese Reaktion besitzt infolgedessen eine höhere Geschwindigkeitskonstante als bei Verwendung eines festphasengebundenen ersten Rezeptors. Da die Antikörperverluste bei der Fixierung an die feste Phase vermieden werden, kann die Menge an erstem Rezeptor weiter verringert werden.

Es ist auch im Gegensatz zu dem in Clin. Chem. 27/6 (1981) beschriebenen Verfahren nicht erforderlich, den ersten und den dritten Rezeptor aus zwei unterschiedlichen Tierspezies zu gewinnen, die noch dazu so gewählt werden müssen, daß Rezeptor 2 nur mit Rezeptor 1 reagiert, nicht aber mit Rezeptor 3.

Die Gewinnung des Antikörpers für den Rezeptor 1 und für den Rezeptor 3 aus der gleichen Tierspezies ermöglicht es schließlich auch, zur Antiserumgewinnung Großtiere heranzuziehen.

Die oben geschilderten Vorteile gelten auch bei der Ausführung des erfindungsgemäßen Verfahrens mit nur einer Inkubationsstufe. Im letzteren Falle lassen sich allerdings nicht ebenso hohe Spezifitäten erzielen als bei der bevorzugten Ausführungsform mit zwei Inkubationsschritten.

Erfindungsgemäß wird schließlich auch die Empfindlichkeit gesteigert. So wurde für TSH eine Empfindlichkeit von weniger als 40 pg/ml (1 $\mu U \hat{=} 170$ pg/ml) gefunden, während sie beim 2 site Assay (Sandwichverfahren) über 100 pg/ml liegt.

Die folgenden Beispiele erläutern die Erfindung weiter :

Beispiel 1

Bestimmung von Thyreotropin (TSH)

In Plastikröhrchen, die adsorptiv mit ca. 100 ng anti-Kaninchen-FcY vom Schaf, erhalten durch Immunisierung mit FcY-Fragmenten aus Kaninchen IgG, beschichtet sind (Beschichtungsverfahren entspricht üblichen bekannten Methoden), werden 200 $\mu l$ einer Probe oder der Standards, enthaltend steigende oder unbekannte Mengen an Thyreotropin und 1 000 $\mu l$ einer Lösung bestehend aus Puffer A und 20 ng/ml von anti-TSH-Antikörpern vom Kaninchen pipettiert. (Puffer A : 10 mM $NaH_2PO_4/KH_2PO_4$ pH 7,0).

Die Lösung wird 12 bis 16 Stunden bei Raumtemperatur inkubiert ; danach wird der Röhrcheninhalt ausgesaugt, einmal mit 0,9 %iger NaCl-Lösung gewaschen und anschließend werden 1 000 $\mu l$ Konjugatlösung, bestehend aus einer wässrigen Lösung von anti-TSH-Antikörpern vom Schaf, an welche Peroxidase kovalent gebunden ist in einer POD-Konzentration von 67 mU/ml, in die Röhrchen pipettieren.

Nach 2 Stunden Inkubationsdauer bei Raumtemperatur wird der Röhrcheninhalt ausgesaugt, gewaschen und anschließend 1 000 $\mu l$ Reagenz zum Nachweis von Peroxydaseaktivität in die Röhrchen pipettiert.

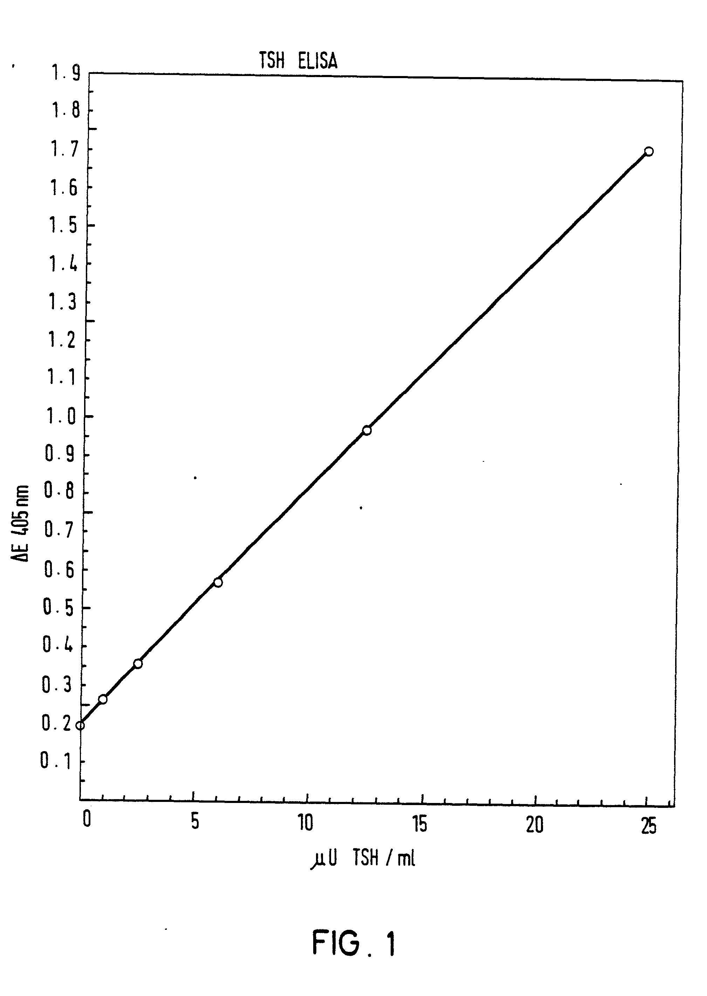

Als Reagenz wird ABTS (1,8 mM, Perborat 3,3 mM in Phosphat-Citrat-Puffer, 100 mM pH 4,4) verwendet. Nach einer weiteren einstündigen Inkubationszeit bei Raumtemperatur wird der Inhalt in eine Küvette überführt und die Extinktion bei 405 nm gegen Reagenz als Leerwert gemessen. Aus den erhaltenen Extinktionen wird die in Fig. 1 dargestellte Eichkurve erstellt.

Die kovalente Bindung (Kopplung) von Peroxydase an Antikörper erfolgt nach der Methode von M. B. Wilson, Paul K. Nakane, beschrieben in « Recent Developments in the Perjodate Methode of Conjugating Horse Radish Peroxidase (HRPO) to Antibodies ». 1978 Elsevier/North-Holland Biomedical Press Seite 215-224 in « Immunfluorescence and Related Staining Techniques ».

ABTS = 2,2'-Azinodi[3-äthyl-benzthiazolin-sulfonat (6)].

Beispiel 2

Bestimmung von $\alpha 1$-Fetoprotein (AFP)

200 $\mu l$ Probe oder Standard enthaltend steigende Mengen an AFP werden mit 30 ng an anti-AFP-Antikörpern vom Kaninchen, gelöst in 1 000 $\mu l$ Phosphat-Puffer, 100 mM, pH 6,8 in dieselben Röhrchen pipettiert, wie sie in Beispiel 1 verwendet werden (enthalten Fc-Fragmente von Kaninchen IgG).

Nach 2 Stunden Inkubation bei Raumtemperatur wird ausgesaugt, gewaschen und 1 000 $\mu l$ Konjugat, bestehend aus anti-AFP-Antikörpern vom Schaf an Peroxydase gekoppelt (nach Nakane) in die Röhrchen pipettiert.

Nach einer weiteren Inkubationsdauer von 2 Stunden bei Raumtemperatur wird erneut ausgesaugt, gewaschen und Substratlösung zum Nachweis der an die Röhrchenwand fixierten Peroxydase-Aktivität einpipettiert. Nach einer einstündigen Inkubationszeit wird bei 405 nm die Adsorption am Photometer gemessen.

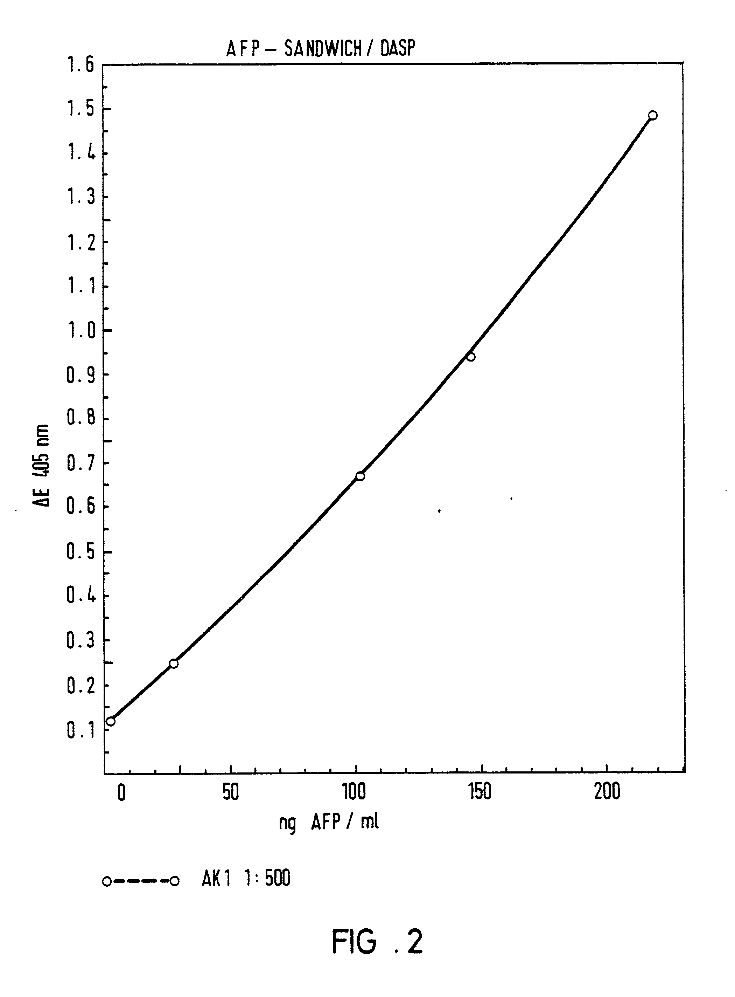

Die Auswertung erfolgt über die in Fig. 2 der Zeichnung dargestellte Eichkurve wie in Beispiel 1 beschrieben.

Beispiel

Bestimmung von $\alpha 1$-Fetoprotein

In dieser Durchführungsform werden Röhrchen eingesetzt, die mit anti-Digoxin-Antikörpern vom Kaninchen adsorptiv (100 ng anti-Digoxin-Antikörper) beladen sind.

Als Antikörper-1 wird ein Konjugat aus Digoxin und anti-AFP-Antikörper vom Schaf (20 ng/ml) verwendet. Die Herstellung des Konjugats erfolgt wie in der DE-Patentschrift 25 37 129 beschrieben.

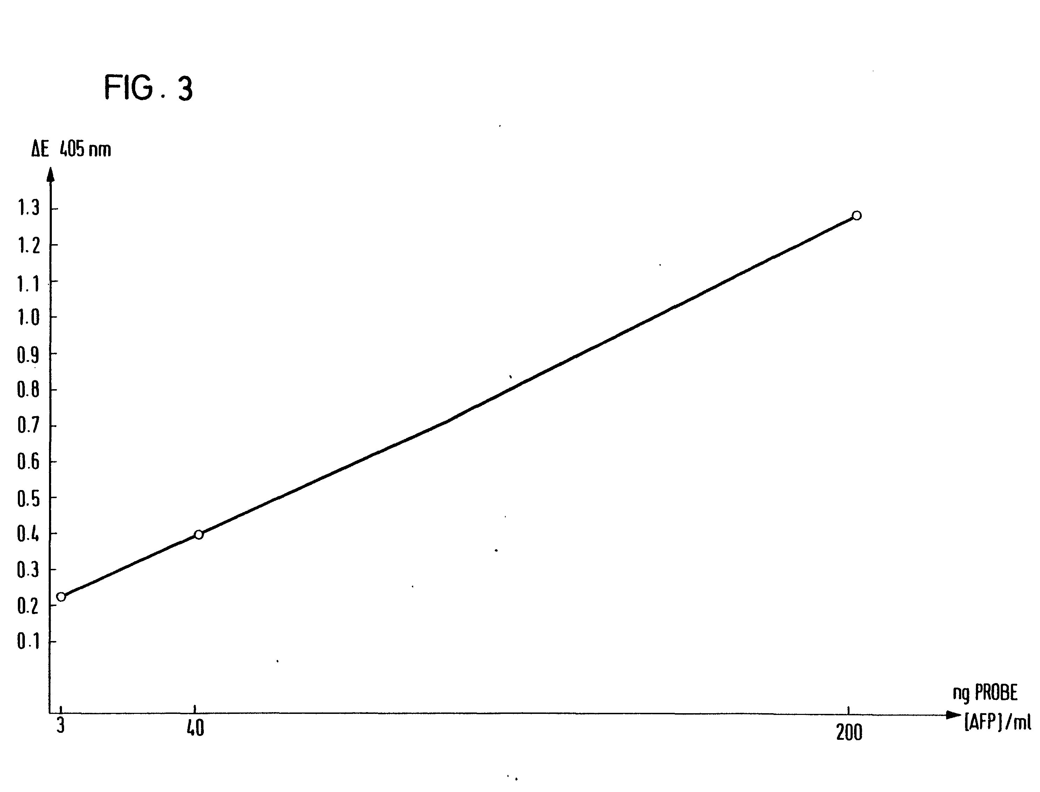

Die Durchführungsform entspricht im übrigen Beispiel 2. Die erhaltene Eichkurve zeigt Fig. 3.

**Patentansprüche**

1. Verfahren zur Bestimmung eines polyvalenten Antigens durch Inkubation mit 3 verschiedenen Rezeptoren, von denen der erste und der dritte in flüssiger Phase vorliegen und mit dem Antigen bindefähig sind, der zweite Rezeptor in fester Phase vorliegt und mit dem ersten Rezeptor bindefähig ist und der dritte Rezeptor eine Markierung trägt und mit dem ersten und zweiten Rezeptor nicht kreuzreagiert, Trennung der festen von der flüssigen Phase, wobei man das Antigen entweder mit allen drei Rezeptoren oder mit dem ersten und zweiten Rezeptor gleichzeitig inkubiert, dann die Phasen trennt, gegebenenfalls die feste Phase mit dem dritten Rezeptor inkubiert, erneut die Phasen trennt und die Markierung in einer der Phasen mißt, dadurch gekennzeichnet, daß man einen ersten und einen dritten Rezeptor verwendet, die jeweils einen Antikörper oder ein Fragment davon enthalten und beide Antikörper bzw. Fragmente aus der gleichen Tierspezies stammen.

2. Verfahren nach Anspruch 1, dadurch gekennzeichnet, daß man als ersten Rezeptor einen Antikörper verwendet.

3. Verfahren nach Anspruch 2, dadurch gekennzeichnet, daß man als ersten Rezeptor einen covalent mit einem Hapten verbundenen Antikörper verwendet.

4. Verfahren nach Anspruch 2, dadurch gekennzeichnet, daß man einen zweiten Rezeptor verwendet, der nur mit einem Teil des ersten Rezeptors bindefähig ist.

5. Verfahren nach Anspruch 3, dadurch gekennzeichnet, daß man einen zweiten Rezeptor verwendet, der mit dem Fc-Teil oder dem Haptenteil des ersten Rezeptors bindefähig ist.

6. Verfahren nach einem der Ansprüche 2 bis 5, dadurch gekennzeichnet, daß man als dritten Rezeptor einen Teil des ersten Rezeptors verwendet.

7. Verfahren nach Anspruch 6, dadurch gekennzeichnet, daß man einen dritten Rezeptor verwendet, welcher ein Fab-Fragment des ersten Rezeptors ist.

8. Verfahren nach einem der vorhergehenden Ansprüche, dadurch gekennzeichnet, daß man als zweiten Rezeptor einen Antikörper verwendet, der von der gleichen Tierspezies wie der erste und dritte Rezeptor stammt.

9. Verfahren nach einem der vorhergehenden Ansprüche, dadurch gekennzeichnet, daß man einen dritten Rezeptor verwendet, welcher mit einem Enzym, einer fluoreszierenden, chemilumineszierenden oder radioaktiven Substanz markiert ist.

10. Trockenreagenz zur Bestimmung eines polyvalenten Antigens nach einem der vorhergehenden Ansprüche, welches zwei verschiedene lösliche, mit dem Antigen bindefähige Rezeptoren, von denen einer eine Markierung trägt und einen mit dem nicht markierten löslichen Rezeptor bindefähigen unlöslichen Rezeptor enthält, dadurch gekennzeichnet, daß beide löslichen Rezeptoren Antikörper oder Fragmente davon enthalten, die aus der gleichen Tierspezies stammen, wobei der unlösliche Rezeptor und der unmarkierte lösliche Rezeptor physikalisch getrennt vorliegen.

11. Trockenreagenz nach Anspruch 10, dadurch gekennzeichnet, daß der unlösliche Rezeptor ein Fragment eines Antikörpers enthält, der aus der gleichen Tierspezies wie die Antikörper bzw. Fragmente in den löslichen Rezeptoren stammt.

## Claims

1. Process for the determination of a polyvalent antigen by incubation with 3 different receptors, of which the first and the third are present in liquid phase and are bindable with the antigen, the second receptor is present in solid phase and is bindable with the first receptor and the third receptor carries a labelling and does not cross-react with the first and second receptor, separation of the solid from the liquid phase, whereby one simultaneously incubates the antigen either with all three receptors or with the first and second receptor, then separates the phases, possibly incubates the solid phase with the third receptor, again separates the phases and measures the labelling in one of the phases, characterised in that one uses a first and a third receptor which, in each case, contains an antibody or a fragment thereof and both antibodies or fragments originate from the same animal species.

2. Process according to claim 1, characterised in that one uses an antibody as first receptor.

3. Process according to claim 2, characterised in that one uses an antibody covalently bound with a hapten as first receptor.

4. Process according to claim 2, characterised in that one uses a second receptor which is only bindable with a part of the first receptor.

5. Process according to claim 3, characterised in that one uses a second receptor which is bindable with the Fc part or the hapten part of the first receptor.

6. Process according to one of claims 2 to 5, characterised in that as third receptor one uses a part of the first receptor.

7. Process according to claim 6, characterised in that one uses a third receptor which is a Fab fragment of the first receptor.

8. Process according to one of the preceding claims, characterised in that as second receptor one uses an antibody which originates from the same animal species as the first and third receptor.

9. Process according to one of the preceding claims, characterised in that one uses a third receptor which is labelled with an enzyme, a fluorescing, chemiluminescing or radioactive substance.

10. Dry reagent for the determination of a polyvalent antigen according to one of the preceding claims, which contains two different soluble receptors bindable with the antigen, of which one carries a labelling, and an insoluble receptor bindable with the non-labelled soluble receptor, characterised in that both soluble receptors contain antibodies or fragments thereof which originate from the same animal species, whereby the insoluble receptor and the unlabelled soluble receptor are present physically separated.

11. Dry reagent according to claim 10, characterised in that the insoluble receptor contains a fragment of an antibody which originates from the same animal species as the antibodies or fragments in the soluble receptors.

## Revendications

1. Procédé de dosage d'un antigène polyvalent par incubation avec 3 récepteurs différents, parmi lesquels les premier et troisième sont présents en phase liquide et susceptibles de se lier à l'antigène, le deuxième récepteur se trouve en phase solide et est susceptible de se lier au premier récepteur et le troisième récepteur est porteur d'un marquage et n'entre pas en réaction croisée avec les premier et deuxième récepteurs, par séparation de la phase solide de la phase liquide, moyennant quoi on incube l'antigène simultanément avec tous les trois récepteurs ou avec les premier et deuxième récepteurs, sépare ensuite les phases, incube le cas échéant la phase solide avec le troisième récepteur, sépare à nouveau les phases et mesure le marquage dans une des phases, caractérisé en ce qu'on utilise un premier et un troisième récepteurs qui contiennent dans chaque cas un anticorps ou un fragment de celui-ci et que les deux anticorps ou fragments proviennent de la même espèce animale.

2. Procédé selon la revendication 1, caractérisé en ce qu'on utilise un anticorps comme premier récepteur.

3. Procédé selon la revendication 2, caractérisé en ce qu'on utilise comme premier récepteur un anticorps lié de façon covalente à un haptène.

4. Procédé selon la revendication 2, caractérisé en ce qu'on utilise un deuxième récepteur qui n'est susceptible de se lier qu'avec une partie du premier récepteur.

5. Procédé selon la revendication 3, caractérisé en ce qu'on utilise un deuxième récepteur susceptible de se lier avec la partie Fc ou la partie haptène du premier récepteur.

6. Procédé selon l'une quelconque des revendications 2 à 5, caractérisé en ce qu'on utilise comme troisième récepteur une partie du premier récepteur.

7. Procédé selon la revendication 6, caractérisé en ce qu'on utilise un troisième récepteur qui est un fragment Fab du premier récepteur.

8. Procédé selon l'une des revendications précédentes, caractérisé en ce qu'on utilise comme deuxième récepteur un anticorps qui provient de la même espèce animale que les premier et troisième récepteurs.

9. Procédé selon l'une des revendications précédentes, caractérisé en ce qu'on utilise un troisième récepteur qui est marqué par une enzyme, par une substance fluorescente, chimiolumines-cente ou radioactive.

10. Réactif sec pour le dosage d'un antigène polyvalent selon l'une des revendications précédentes, qui contient deux récepteurs solubles différents susceptibles de se lier à l'antigène, dont l'un porte un marquage et contient un récepteur insoluble susceptible de se lier au récepteur soluble non marqué, caractérisé en ce que les deux récepteurs solubles contiennent des anticorps ou des fragments de ceux-ci provenant de la même espèce animale, le récepteur insoluble et le récepteur soluble non marqué se trouvant physiquement séparés.

11. Réactif sec selon la revendication 10, caractérisé en ce que le récepteur insoluble contient un fragment d'un anticorps qui provient de la même espèce animale que les anticorps, ou les fragments dans les récepteurs solubles.

TSH ELISA

FIG. 1

FIG . 2

FIG. 3